(19) Europäisches Patentamt
European Patent Office
Office européen des brevets

(11) **EP 2 442 105 B1**

(12) **EUROPEAN PATENT SPECIFICATION**

(45) Date of publication and mention of the grant of the patent:
**07.01.2015 Bulletin 2015/02**

(51) Int Cl.:
**G01N 33/68** (2006.01)

(21) Application number: **10785954.8**

(22) Date of filing: **09.06.2010**

(86) International application number:
**PCT/JP2010/003836**

(87) International publication number:
**WO 2010/143422 (16.12.2010 Gazette 2010/50)**

(54) **Test method on renal diseases**

Testverfahren für Nierenerkrankungen

Méthode permettant de déceler des affections rénales

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **10.06.2009 JP 2009139187**

(43) Date of publication of application:
**18.04.2012 Bulletin 2012/16**

(73) Proprietors:
• **Niigata University**
**Niigata 950-2181 (JP)**
• **Juntendo Educational Foundation**
**Tokyo 113-8421 (JP)**
• **Denka Seiken Co., Ltd.**
**Chuo-ku, Tokyo 103-0025 (JP)**
• **Hara, Masanori**
**Niigata 950-2041 (JP)**

(72) Inventors:
• **HARA, Masanori**
**Niigata-shi**
**Niigata 950-2041 (JP)**
• **SAITO, Akihiko**
**Niigata-shi**
**Niigata 951-8510 (JP)**
• **TOMINO, Yasuhiko**
**Tokyo 113-8421 (JP)**
• **ASANUMA, Katsuhiko**
**Tokyo 113-8421 (JP)**
• **KUROSAWA, Hiroyuki**
**Gosen-shi**
**Niigata 959-1695 (JP)**

• **OGASAWARA, Shinya**
**Gosen-shi**
**Niigata 959-1695 (JP)**
• **HIRAYAMA, Yoshiaki**
**Gosen-shi**
**Niigata 959-1695 (JP)**

(74) Representative: **Zwicker, Jörk et al**
**ZSP Patentanwälte**
**Partnerschaftsgesellschaft**
**Radlkoferstrasse 2**
**81373 München (DE)**

(56) References cited:
EP-A- 1 336 845      WO-A1-2011/105474
JP-A- 6 011 507      JP-A- 2008 122 410
JP-A- 2008 122 410      JP-A- 2008 185 536
JP-A- 2008 185 536

• SHIGERU SEKINE ET AL.: 'Jin Shogai no Atarashii Marker ni Tsuite -Nyochu Podocyte, Podocalyxin to Rinsho Byorigakuteki Igi-' THER.RES. vol. 29, no. 11, 2008, pages 1900 - 1904, XP008148139
• Richard J Glassock ET AL: "Ageing and the glomerular filtration rate: truths and consequences", Transactions of the American Clinical and Climatological Association, 1 January 2009 (2009-01-01), pages 419-428, XP055099917, United States Retrieved from the Internet: URL:http://www.ncbi.nlm.nih.gov/pubmed/197 68194

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 442 105 B1

**(Cont. next page)**

- M Hara ET AL: "25th Study Group on IgA Nephropathy Urinary sediment podocalyxin in children with IgA nephropathy and Henoch Schoenlein purpura nephritis", Nephrology, Volume7, Issue Supplement S3, 28 June 2008 (2008-06-28), page A109, XP055100040, DOI: 10.1111/j.1440-1797.2002.tb00545. Retrieved from the Internet: URL:http://onlinelibrary.wiley.com/store/1 0.1111/j.1440-1797.2002.tb00545.x/asset/j.1440-1797.2002.tb00545.x.pdf?v=1&t=hr8tt6b 6&s=7f48723a9c6303a7184cb444d0b67e069958 7e 81 [retrieved on 2014-02-04]

- R. J. GLASSOCK ET AL: "IgA nephropathy: Challenges and opportunities", CLEVELAND CLINIC JOURNAL OF MEDICINE, vol. 75, no. 8, 1 August 2008 (2008-08-01) , pages 569-576, XP055100043, ISSN: 0891-1150, DOI: 10.3949/ccjm.75.8.569

Remarks:
    The file contains technical information submitted after the application was filed and not included in this specification

**Description**

Technical Field

The present invention relates to a test method for a renal disease, including using urinary podocalyxin and at least one or more additional markers in combination.

Background Art

In recent years, the number of patients with renal diseases has been increasing year by year. The causes and progression of the renal diseases are not uniform among the patients. In some cases, renal injuries are caused by lesions at sites other than the kidney such as diabetes, resulting in renal failures. In other cases, the renal diseases are caused by, for example, primary glomerulonephritis such as IgA nephropathy.

A test for a urine protein or a urine sediment through the so-called urinalysis has widely been carried out as a clue to diagnose the renal diseases. However, even in a healthy subject, the urine protein may transiently increase, for example, after excessive exercise, under psychological stress, during fever, in consuming a large amount of meat, or before menstruation. Further, some urine proteins, such as orthostatic proteinuria, are not derived from the renal diseases. The test for the urine sediment includes testing urinary tract bleeding by centrifuging urine and observing an increase in erythrocytes in the sediment with a microscope. However, the test with the urine sediment cannot necessarily assess urinary tract bleeding without fail because the erythrocytes are observed in urine even in a healthy subject and the bleeding is not derived from the renal injuries but derived from urinary tract system-related organs in some cases. In addition, serum creatinine (SCr), blood urea nitrogen (BUN), and the like are measured for the purpose of testing the blood retention of a urinary component, but those values are liable to affected, for example, by a meal for a subject to be tested (hereinafter, sometimes referred to as "subject").

Accordingly, it is currently recognized that histological diagnosis with renal biopsy is indispensable for the diagnosis of the renal diseases and the final assessment of the severity thereof. However, the renal biopsy involves collecting part of renal tissues and evaluating the collected part with a microscope, is an invasive test, and hence always has risks of complications such as bleeding and infection. Further, a patient who is to undergo the renal biopsy needs to be hospitalized in a facility with specialists and equipment, and physical and social burdens are imposed on the patient, which are non-negligible.

The renal biopsy is mainly applied in the following cases: 1) a case where a urine protein is found at 1.0 g or more per day; 2) a case where cryptogenic renal injury is found but renal atrophy is not found in an image test; 3) a case where hematuria is continuously found and there is suspicion of progressive chronic nephritis; or 4) a case where a rapid deterioration of renal function is found. Meanwhile, a case where the renal biopsy is contraindicated is exemplified by the following cases: 1) a case where renal atrophy due to chronic renal dysfunction has already been found in an image test; 2) a case where it is difficult to stop bleeding because of a bleeding tendency or out-of-control hypertension; 3) a case with polycystic kidney; or 4) a patient who cannot keep quiet during renal biopsy and during and after a test or a case where instructions are not followed. There are very many cases where the renal biopsy described above cannot be conducted in actual clinical fields, which is problematic.

There is disclosed a simple test measure for renal injury involving measuring urinary podocalyxin as a substance found in association with renal diseases (Patent Literature1). Podocalyxin is a sugar protein which is present in the surface of podocytes constructing the renal glomerulus and is responsible for a filtration function. The podocytes are located on the Bowman's space side in the glomerular basement membrane and play important roles in the mechanism of glomerular filtration. Thus, it is known that the grasping of the degree of damage in the podocytes has an extremely important meaning in understanding renal diseases (Non Patent Literature 1).

In the renal diseases, IgA nephropathy (Berger's disease) is one of the primary chronic glomerulonephritides and accounts for about 40% or more of the diseases. It is known that IgA nephropathy is a poor prognostic disease whose symptoms are not improved by dosing in about 30% of patients with the disease and which requires dialysis for end-stage renal failure at a later time. At present, when the subject has findings suspected of having IgA nephropathy through a urine test and a blood test, the subject is subjected to renal biopsy. As a result, the definitive diagnosis of IgA nephropathy is performed and prognostic classification is performed. IgA nephropathy can be classified into four groups, i.e., a good prognosis group, a relatively good prognosis group, a relatively poor prognosis group, and a poor prognosis group. The classification is based on the definitive diagnosis using the renal biopsy. The four groups are described below.

1) Good prognosis group: dialysis is very unlikely to be required; as glomerular findings, only mild mesangial cell proliferation and increased matrix are found, and no glomerulosclerosis, crescent formation, and adhesion to the Bowman's capsule are found; and as renal tubular, interstitial, and vascular findings, remarkable renal tubular, interstitial, and vascular changes are not found.

2) Relatively good prognosis group: dialysis is unlikely to be required; as glomerular findings, mild mesangial cell proliferation and increased matrix are found, andglomerulosclerosis, crescent formation, and adhesion to the Bowman's capsule are found in less than 10% of all the glomeruli subjected to biopsy; and as renal tubular,interstitial,and vascularfindings,remarkable renal tubular, interstitial, and vascular changes are not found.

3) Relatively poor prognosis group: dialysis is likely to be required within 5 or more to 20 or less years; as glomerular findings, moderate mesangial cell proliferation and increased matrix are found, and glomerulosclerosis, crescent formation, and adhesion to the Bowman's capsule are found in 10 to 30% of all the glomeruli subj ected to biopsy; and as renal tubular, interstitial, and vascular findings, renal tubular atrophy is mild, cell infiltration is mild in the interstitium except around some sclerosed glomeruli, and a mild sclerosis change is found in the blood vessel.

4) Poor prognosis group: dialysis is likely to be required within 5 years; as glomerular findings, severe mesangial cell proliferation and increased matrix are found, and glomerulosclerosis, crescent formation, and adhesion to the Bowman's capsule are found in 30% or more of all the glomeruli subjected to biopsy; when sites of sclerosis are totaled and converted to global sclerosis, the sclerosis rate is 50% or more of all the glomeruli; some glomeruli also show compensatory hypertrophy; as renal tubular, interstitial, and vascular findings, renal tubular atrophy and interstitial cell infiltration are severe, and fibrosis is also severe; and thickening or degeneration is found in some intrarenal arteriole walls.

For IgA nephropathy, the proportion of the primary disease of chronic kidney disease (CKD) is the highest, and the disease is found as asymptomatic hematuria or proteinuria in school urinalysis, office examination, or the like in many cases. In adult humans, IgA nephropathy is a major cause for chronic renal failure. Regarding the diagnosis of IgA nephropathy, there is proposed, for example, a method involving immunologically detecting immunoglobulin A (IgA) in serum from a subject (Patent Literatures 2 and 3). Also, urinary sediment podocalyxin has been suggested as reliable and useful laboratory marker for the estimation of the severity of acute glomerular injury and a urinary index of acute extracapillary changes in children with IgA nephropathy and Henoch Schoenlein purpura nephrits (Non Patent Literature 2)

Citation List

Patent Literature

[PTL 1] WO 2002/037099 A1
[PTL 2] JP 2592121 B2
[PTL 3] JP 2000-241431 A

Non Patent Literature

[NPL 1] Hara et al., Nephron 69: 397-403 (1995)
[NPL 2] Hara et al., Nephrology, Volume 7, Issue Suppl S3, Alog (28 June 2008)

Summary of Invention

Technical Problem

An object of the present invention is to provide a test method for a prognostic prediction of IgA nephropathy in a subject suspected of having a renal disease.

Solution to Problem

In order to solve the above-mentioned problem, the inventors of the present invention have focused on the clinical significance of urinary podocalyxin in the assessment of a renal disease, and have found that the use of urinary podocalyxin and at least one additional marker in combination allows the renal disease to be effectively tested. Thus, the present invention has been completed. The present invention is defined in its broadest sense by the appended claims.

That is, the present invention includes the following items.

1. A test method for prognostic prediction of IgA nephropathy, comprising calculating a podocalyxin index value using a value for the urinary podocalyxin and a value for the additional marker, and using the podocalyxin index value as an indicator, wherein the podocalyxin index value is obtained

by dividing the urinary podocalyxin excretion rate (PCX/Cre) by the estimated glomerular filtration rate (eGFR) or
by multiplying the urinary podocalyxin excretion rate (PCX/Cre) by the value for the urine protein excretion rate (urine protein/Cre).

2. A test method for prognostic prediction of IgA nephropathy according to claim 1, further comprising assessing a subject to be tested who has a podocalyxin index value higher than a podocalyxin reference value to be in need of renal biopsy.

3. A test method for prognostic prediction of IgA nephropathy according to claim 2, wherein the podocalyxin reference value comprises a value obtained from a group of subjects to be tested including a good prognosis group and a relatively good prognosis group of IgA nephropathy,
wherein a subject from a good prognosis group is defined

in that no glomerulosclerosis, crescent formation, and adhesion to the Bowman's capsule are found and
in that remarkable renal tubular, interstitial, and vascular changes are not found; and

wherein a subject from a relatively good prognosis group is defined

in that glomerulosclerosis, crescent formation, and adhesion to the Bowman's capsule are found in less than 10% of all the glomeruli subjected to biopsy and in that remarkable renal tubular, interstitial, and vascular changes are not found.

4. A test method for prognostic prediction of IgA nephropathy according to any one of claims 1 to 3, wherein the test using urinary podocalyxin is performed on specimens, with the exception of specimens that had been assessed to belong to a poor prognosis group with an additional renal function marker, wherein a subject from a poor prognosis group is defined

in that glomerulosclerosis, crescent formation, and adhesion to the Bowman's capsule are found in 30% or more of all the glomeruli subjected to biopsy and
in that the sclerosis rate is 50% or more of all the glomeruli, when sites of sclerosis are totaled and converted to global sclerosis.

5. A test method for prognostic prediction of IgA nephropathy according to any one of claims 1 to 4, wherein the detecting of the urinary podocalyxin is carried out by an immunological technique.

Advantageous Effects of Invention

The test method of the present invention allows the discrimination of a poor prognosis group for not only cases with overt findings showing poor prognosis in a conventional test method but also for cases with no overt findings, and thus allows the assessment of a renal disease to be performed more exactly. Further, in the test method of the present invention, also in the cases with overt findings, the assessment of a renal disease can be effectively performed. For example, by virtue of the test method of the present invention, definitive diagnosis can be performed by further performing renal biopsy in a subject predicted to belong to a relatively poor prognosis group or a poor prognosis group of IgA nephropathy. By virtue of the test method of the present invention, it is not necessary to perform renal biopsy in a patient with a mild renal disease, which allows physical and social burdens on a subject to be reduced and allows a therapeutic strategy or the like to be rapidly decided.

Brief Description of Drawings

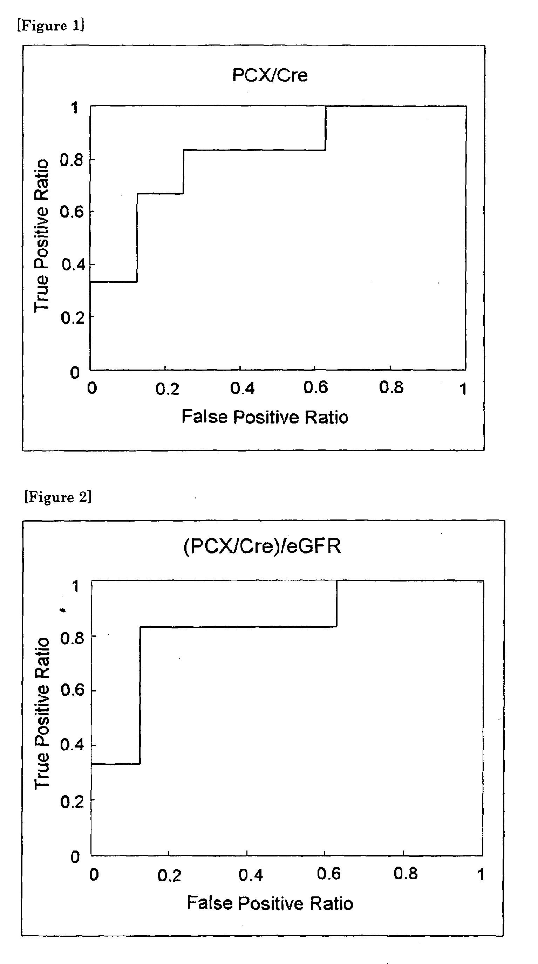

[FIG. 1] FIG. 1 is a graph illustrating, for patients with IgA nephropathy with an estimated glomerular filtration rate eGFR of $\geq 60$ and a urine protein excretion rate "urine protein/Cre" of less than 0.5 g/g of creatinine, a prognostic screening effect (ROC curve) using as an indicator a urinary podocalyxin excretion rate "PCX/Cre" (Example 2).

[FIG. 2] FIG. 2 is a graph illustrating, for patients with IgA nephropathy with an estimated glomerular filtration rate eGFR of ≥60 and a urine protein excretion rate "urine protein/Cre" of less than 0.5 g/g of creatinine, a prognostic screening effect (ROC curve) using as an indicator an index value "(PCX/Cre) /eGFR" obtained by dividing a urinary podocalyxin excretion rate "PCX/Cre" by eGFR (Example 2).

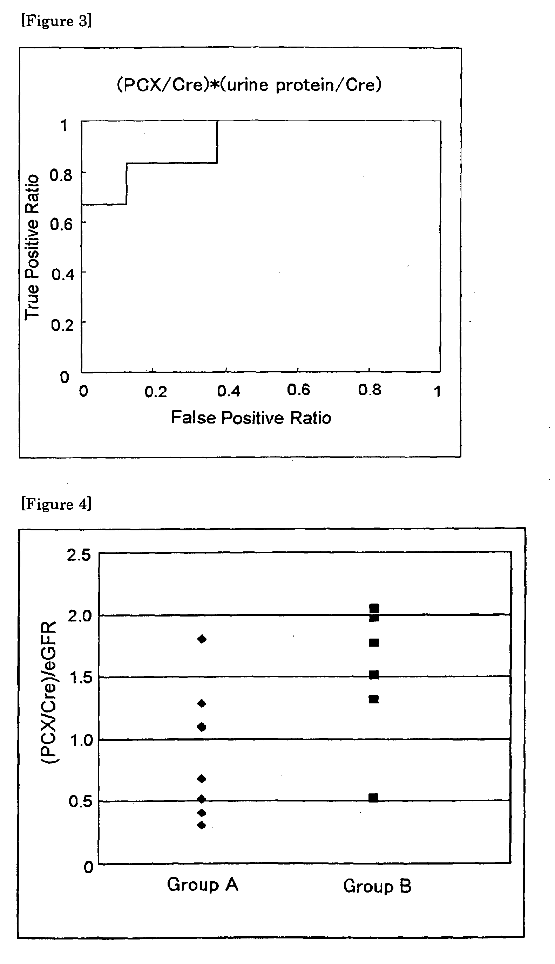

[FIG. 3] FIG. 3 is a graph illustrating, for patients with IgA nephropathy with an estimated glomerular filtration rate eGFR of ≥60 and a urine protein excretion rate "urine protein/Cre" of less than 0.5 g/g of creatinine, a prognostic screening effect (ROC curve) using as an indicator an index value "(PCX/Cre) * (urine protein/Cre) " obtained by multiplying a urinary podocalyxin excretion rate "PCX/Cre" by a urine protein excretion rate "urine protein/Cre" (Example 2).

[FIG. 4] FIG. 4 is a graph illustrating, for patients with IgA nephropathy with an estimated glomerular filtration rate eGFR of ≥60 and a urine protein excretion rate "urine protein/Cre" of less than 0.5 g/g of creatinine, a comparison of index values "(PCX/Cre)/eGFR" each obtained by dividing a urinary podocalyxin excretion rate "PCX/Cre" by eGFR in accordance with prognostic classification based on histological findings of renal biopsy (Example 2).

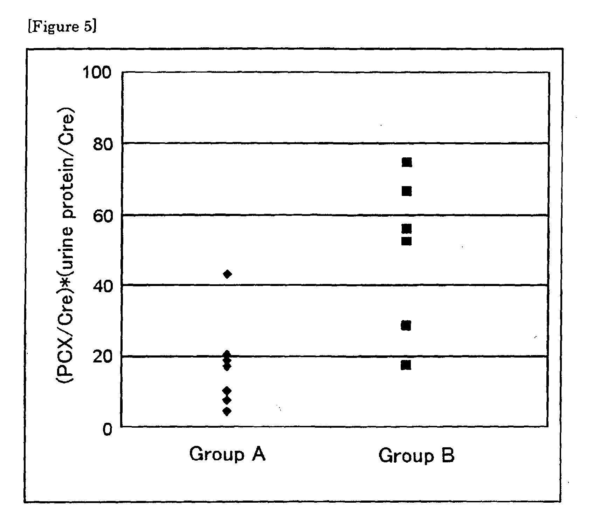

[FIG. 5] FIG. 5 is a graph illustrating, for patients with IgA nephropathy with an estimated glomerular filtration rate eGFR of ≥60 and a urine protein excretion rate "urine protein/Cre" of less than 0.5 g/g of creatinine, a comparison of index values "(PCX/Cre)*(urine protein/Cre)" each obtained by multiplying a urinary podocalyxin excretion rate "PCX/Cre" by a urine protein excretion rate in accordance with prognostic classification based on histological findings of renal biopsy (Example 2).

Description of Embodiments

The present invention is a test method for prognostic prediction of IgA nephropathy, including detecting urinary podocalyxin in a subject and using the urinary podocalyxin and one or more additional markers in combination in a podocalyxin index value.

In this description, urine as a specimen may be derived from any subject. No particular limitation is imposed on a collection method for urine, but it is preferred to use early morning urine or casual urine. Further, the amount of urine necessary for the test method of the present invention is about 10 to 200 μL. The test method of the present invention may be performed concurrently with, for example, a general urine test to be conventionally performed in a medical examination or the like. Alternatively, the test method may be performed using urine collected separately from a subject judged to be suspected of having a renal disease in a test other than renal biopsy.

Examples of the renal disease include various diseases such as chronic kidney disease (CKD) and acute renal failure, and also include diabetic nephropathy, membranous nephropathy, focal glomerulosclerosis, membranoproliferative glomerulonephritis, lupus nephritis, and IgA nephropathy. Preferred examples thereof include IgA nephropathy. IgA nephropathy is a disease **characterized in that** glomerular mesangial cell proliferation, enlarged (increased) mesangial matrix, and granular deposits mainly formed of IgA in a mesangial region are found in chronic glomerulonephritis. IgA nephropathy can be diagnosed in accordance with the guidelines for diagnosis with a urine test and a blood test provided in 1995 by a "joint committee of the Special Study Group on Progressive Glomerular Disease, Ministry of Health and Welfare of Japan and the Japanese Society of Nephrology." In the urine test, a subject with continuous microscopic hematuria or intermittent or continuous proteinuria is judged to be suspected of having IgA nephropathy. In the blood test, a subject with a serum IgA value of 315 mg/dL or more is judged to be suspected of having IgA nephropathy.

Urine as a specimen may be treated by adding and mixing a treatment liquid into the collected urine. The treatment liquid may be any as long as the pH adjustment of the urine, the masking of a urine sediment, and the solubilization of podocalyxin are possible, but is preferably exemplified by a solution obtained by adding a chelating agent, a surfactant, and the like to a buffer. The buffer and the chelating agent may be any known buffer and chelating agent, and it is preferred to use a nonionic surfactant as the surfactant. The treatment liquid is exemplified by a solution including 0.2 M EDTA and 2% (Vol./Vol.) Triton X-100 in 2 M TES-NaOH (pH 7.0). A urine sample solution can be obtained by adding and mixing 10 μL of such treatment liquid into 90 μL of a urine specimen.

Various methods may be employed as a detection method for podocalyxin in the urine sample solution. An example of the detection method for the urinary podocalyxin is an immunological technique. The immunological technique may be performed, for example, by an immunostaining method (including a fluorescent antibody method, an enzymatic antibody method, a heavy metal-labeled antibody method, and a radioisotope-labeled antibody method), a combination of separation based on an electrophoresis method and a detection method with fluorescence, an enzyme, a radioisotope, or the like (including a western blot method and a fluorescent two-dimensional electrophoresis method), enzyme-linked immunosorbent assay (ELISA), a dot blotting method, latex agglutination-turbidimetric immunoassay (LA), or immunochromatography. Of those, it is preferred to employ an ELISA method or an LA method. It is preferred to employ a sandwich method in the ELISA method from the viewpoint of quantitative property. In the sandwich method, a urine sample solution is added to an anti-podocalyxin antibody-coated microtiter plate to cause an antigen-antibody reaction, an enzyme-labeled anti-podocalyxin antibody is further added to cause an antigen-antibody reaction, the plate is washed and then subjected to a reaction with an enzyme substrate and color development, the absorbance is measured to detect urinary podocalyxin, and the measured value can be used to calculate a urinary podocalyxin concentration.

The anti-podocalyxin antibody for use in the immunological technique has only to be an antibody capable of detecting podocalyxin. The anti-podocalyxin antibody for use in the present invention is not particularly limited, and may be a known antibody or an antibody to be developed in the future. Examples thereof include monoclonal and polyclonal antibodies, a labeled antibody, a chimeric antibody, a humanized antibody, and binding active fragments thereof.

A value for urinary podocalyxin for use in the present invention is a urinary podocalyxin concentration corrected with a value for a urinary component to be stably excreted in urine (urinary component value). The urinary component is particularly preferably urinary creatinine. It is considered that urinary creatinine is substantially constant irrespective of a disease in one individual because the production of creatinine depends on the amount of a muscle. In a test for a urinary excretion substance, in order to eliminate an error in urinary amount, a technique involving correcting the amount of a urinary excretion substance of interest with an amount per g of creatinine is generally employed. This allows the comparison of urinary excretion substances per unit gram of creatinine. A corrected value obtained by correcting a urinary podocalyxin concentration with a urinary creatinine concentration is referred to as urinary podocalyxin excretion rate (PCX/Cre), and the urinary podocalyxin excretion rate can be calculated with the following equation.

```
<Equation>  PCX/Cre:  urinary  podocalyxin  excretion  rate

(μg/g)=100×urinary  podocalyxin  concentration  (ng/mL)/urinary

creatinine concentration (mg/dL)
```

In the present invention, various markers may be used as an additional marker other than urinary podocalyxin to be used in combination with the urinary podocalyxin. The additional marker is a marker representing a living body function, and may be one which has already been clinically used as a known marker, or may be one newly found. Any marker may be used as long as a renal disease can be tested with a combination thereof with the urinary podocalyxin. Further, the additional marker may be corrected with a value for a urinary component to be stably excreted in urine. The additional marker is mainly exemplified by a renal function marker. Examples of the additional renal function marker include, but not limited to, an estimated glomerular filtration rate (eGFR) andaurineprotein. In the test method, the use of urinary podocalyxin and an additional marker other than the urinary podocalyxin in combination means, for example, obtaining a new podocalyxin index value (hereinafter, simply referred to as "index value") calculated using a value for the urinary podocalyxin and a value for the additional marker and/or preliminarily performing the assessment of a renal disease with the additional marker and then performing the assessment with the urinary podocalyxin.

In the present invention, the index value is used as an indicator. The index value is obtained by multiplying or dividing a urinary podocalyxin excretion rate by a value for the additional renal function markers eGFR or urine protein excretion rate. It is conceivable that such index value reflects the status of active disease progression of glomerular lesions in real time.

An estimated glomerular filtration rate (eGFR) as one of the additional renal function markers is a calculated value for a glomerular filtration rate (GFR) as the rate of urine which can be treated per minute in the kidney. The estimated glomerular filtration rate may be calculated, for example, with an age, a gender, and a serum creatinine concentration. As the calculation equation for determining the estimated glomerular filtration rate, a known equation or a new equation to be developed in the future may be used, and it is preferred to select and use an appropriate equation depending on attributes of a subject (for example, race and the like). For example, when the subject is Japanese, the estimated glomerular filtration rate can be calculated with the following equation.

```
GFR (male)=194×SCre (serum creatinine)^-1.094×age^-0.287

GFR (female)=GRF (male)×0.739
```

In the present invention, it is preferred to use the estimated glomerular filtration rate (eGFR) as the glomerular filtration rate. A urinary podocalyxin excretion rate is divided by eGFR to calculate an index value "(PCX/Cre) /eGFR." The index value "(PCX/Cre) /eGFR" may be calculated with a computer using software. The index value "(PCX/Cre)/eGFR" may be used as an indicator for the test method of the present invention.

Another example of the additional renal function marker is a urine protein, and a value for the urine protein itself may be obtained by a known technique. As the value for the urine protein, it is preferred to use a urine protein excretion rate (urine protein/Cre) preliminarily corrected with a urinary creatinine concentration. The urinary podocalyxin excretion rate (PCX/Cre) preliminarily corrected with the urinary creatinine concentration is multiplied by the urine protein excretion rate (urine protein/Cre) to calculate an index value "(PCX/Cre)*(urine protein/Cre)." The index value "(PCX/Cre) *(urine protein/Cre) "may be calculated with a computer using software. Such index value "(PCX/Cre)*(urine protein/Cre)" is used as an indicator in the test method of the present invention.

In the present invention, the index value obtained as described above is compared to an appropriately set reference value, to thereby perform the assessment of a renal disease, for example, the assessment of the necessity of renal biopsy and/or prognostic prediction. The reference value may be appropriately set, and a value for urinary podocalyxin, preferably an index value in a healthy subject, or a value for urinary podocalyxin, preferably an index value in a patient with a mild renal disease may be used. For example, when the necessity of renal biopsy in a patient suspected of having IgA nephropathy is tested by the test method of the present invention, a value for urinary podocalyxin, preferably "(PCX/Cre)/eGFR" or "(PCX/Cre)* (urine protein/Cre) " obtained from a group of subjects to be tested including a good prognosis group and a relatively good prognosis group of IgA nephropathy may be used as a reference value. With use of such reference value, a subject who has a value for urinary podocalyxin, preferably an index value higher than the reference value can be assessed to be more likely to belong to a relatively poor prognosis group or a poor prognosis group, which allows prognostic prediction. Similarly, a subject who has a value for urinary podocalyxin, preferably an index value higher than the reference value can be assessed to be in need of performing renal biopsy to confirm the pathology more accurately.

Further, a method involving preliminarily performing the assessment of a renal disease with an additional marker and then performing assessment with urinary podocalyxin is exemplified by a method involving performing a test using urinary podocalyxin except for a specimen assessed to belong to a poor prognosis group with an additional renal function marker. A subj ect having a specimen assessed to belong to a poor prognosis group with an additional renal function marker belongs to a poor prognosis group with overt findings. The urinary podocalyxin can further detect a poor prognosis group with no overt findings with the additional renal function marker, and hence has a large clinical significance. The poor prognosis group with overt findings is preferably detected using a renal function marker such as eGFR and/or a urine protein. For example, when a subject corresponds to any one or both of eGFR of less than 60 and a urine protein excretion rate "urine protein/Cre" of 0.5 g/g of creatinine or more, the subject can be assessed to belong to a poor prognosis group.

The present invention also describes a test reagent for a renal disease and a test reagent kit for a renal disease for use in performing a test for a renal disease each including an anti-podocalyxin antibody for detecting urinary podocalyxin. The anti-podocalyxin antibody included in the test reagent or the test reagent kit may be labeled, for example, with an enzyme or the like. Further, the test reagent kit may include two or more kinds of anti-podocalyxin antibodies and the antibodies are preferably antibodies recognizing epitopes different from each other. In addition, the kit may include a reagent such as a treatment liquid or a chromogenic substrate, an instrument necessary for a test, and the like.

Examples

(Example 1) Measurement of urinary podocalyxin concentration

A podocalyxin concentration was measured using two kinds of anti-human podocalyxin monoclonal antibodies. Those two kinds of antibodies recognize different two epitopes of human podocalyxin, respectively, and are an anti-human podocalyxin monoclonal antibody a (hereinafter, simply referred to as "antibody a") and an anti-human podocalyxin monoclonal antibody b (hereinafter, simply referred to as "antibody b"), respectively. In this example, an antibody a-coated microtiter plate (split type micro plate GF8 high: Nunc) and a horseradish peroxidase (hereinafter, abbreviated as "HRP")-labeled antibody b were used.

First, 90 $\mu$L of primitive urine obtained from a subject were mixed with 10 $\mu$L of a solution of 2 M TES-NaOH, 0.2 M EDTA, and 2% (Vol./Vol.) Triton X-100, pH 7.0. 100 $\mu$L of a urine sample solution obtained by the mixing were added to wells of an antibody a-coated microtiter plate. The plate was left to stand still at 37°C for 1 hour, and the urine sample solution was then removed by decantation from the wells. Washing was performed by adding 3.6 mM $Na_2HPO_4$, 1.4 mM $KH_2PO_4$, 145 mM NaCl, and 0.05% (Vol./Vol.) Tween 20 (hereinafter, abbreviated as "PBS-T") to the wells of the microtiter plate at 200 $\mu$L/well and removing PBS-T by decantation. The washing step was performed a total of three times. After that, an HRP-labeled antibody b solution was added at 100 $\mu$L/well. The plate was left to stand still at 37°C for 1 hour, and the HRP-labeled antibody b solution was then removed by decantation. Washing was performed by adding PBS-T at 200 $\mu$L/well and removing PBS-T by decantation. The washing step was performed a total of three times. After that, a TMB One-Step Substrate System (Dako) was used as a substrate solution for an HRP enzymatic reaction and added at 100 μL/well, and the plate was left to stand still under a light-shielding condition at 25°C for 30 minutes. After that, a 313 mM $H_2SO_4$ solution was added at 100 μL/well as a reaction terminating solution, and each of the wells was measured for its absorbances at wavelengths of 450 nm and 630 nm using Multiskan Ascent and Ascent Software for Multiskan (Dainippon Pharmaceutical Co., Ltd.). Then, a value obtained by subtracting the absorbance at a wavelength of 630 nm from the absorbance at a wavelength of 450 nm was defined as a measured value. Native human podocalyxin extracted from the kidney was used as a standard for a calibration curve to derive a podocalyxin concentration in a specimen. With use of the following equation, a urinary podocalyxin concentration corrected with creatinine was calculated with the following equation.

<Equation> Urinary podocalyxin excretion rate (μg/g)=100×urinary podocalyxin concentration (ng/mL)/urinary creatinine concentration (mg/dL)

(Example 2) Clinical significance of urinary podocalyxin excretion rate as prognostic screening for IgA nephropathy

Urine specimens obtained from 28 patients with IgA nephropathy were each calculated for its urinary podocalyxin excretion rate (PCX/Cre) by the method of Example 1. Further, the same urine specimens were each measured and calculated for its estimated glomerular filtration rate (eGFR) and urine protein excretion rate (urine protein/Cre). Those rates were combined with the urinary podocalyxin excretion rate to obtain an index value, and the significance of the index value as a prognostic screening marker was examined.

The 28 patients with IgA nephropathy were subjected to renal biopsy and broadly classified into two groups, i.e. , a group (Group A) including a good prognosis group and a relatively good prognosis group and a group (Group B) including a relatively poor prognosis group and a poor prognosis group in accordance with prognostic classification based on histological findings in the renal biopsy. An area under the ROC curve was used as an indicator of a screening effect. The receiver operating characteristic curve (ROC curve) is for use in evaluating the accuracy of a screening test or the like and comparing different test methods, and provides the range of a cut-off point to be selected. The ability of a test to discriminate subjects with and without a certain condition can be visually displayed depending on the selection of the cut-off point. The ROC curve plots a true positive ratio, i.e., sensitivity, in the ordinate axis and a false positive ratio, i.e., (1-specificity) in the abscissa axis as measures. In the case of assessing the superiority or inferiority of different tests, a test having the curve positioned on the upper left side is judged as being more excellent. For example, as compared to the ROC curve of one test method, the curve of the other test method is positioned on the upper left side, the other test method can be judged as having higher accuracy and being more excellent. When screening achieves complete discrimination, one plot is displayed at a right-angle corner on the upper left side, and the area under the ROC curve is evaluated as 1.0. In this example, in order to confirm an effect as a screening marker of prognostic classification, an evaluation was made for accuracy in the case of screening Group A and Group B.

First, Table 1 shows screening effects determined from ROC curves for 28 cases with IgA nephropathy.

[Table 1]

|  | PCX/Cre | 1/eGFR | Urine protein /Cre | (PCX/Cre) /eGFR | (PCX/Cre)* (Urine protein/Cre) |
|---|---|---|---|---|---|
| Area under ROC curve | 0.644 | 0.713 | 0.884 | 0.763 | 0.850 |

The results of Table 1 reveal that "PCX/Cre" alone does not exhibit a prognostic screening effect for IgA nephropathy. On the other hand, the results reveal that "(PCX/Cre)/eGFR" or "(PCX/Cre) * (urine protein/Cre) " is expected to exhibit a prognostic screening effect. However, the prognostic screening effect of such indexvalue differs little from the effect of reciprocal eGFR "1/eGFR" or "urine protein/Cre" alone.

As a factor for the suppression of the prognostic screening effect of the index value, it was considered that a case with advanced glomerulosclerosis or a case with an excess urine protein was included in the 28 cases. In the case with advanced glomerulosclerosis or the case with an excess urine protein, it is conceivable that the expression amount of podocalyxin expressed in the renal glomerulus decreases, and urinary podocalyxin excretion disappears owing to a decrease in gap space on the Bowman's space side which can allow urinary excretion. In fact, in such severe case (case with a urine protein excretion rate of 0.5 g/g of urinary creatinine or more and/or eGFR of less than 60), eGFR lowers, and concurrently the disruption of a renal tubule reabsorption ability occurs, which causes an increase in urine protein. Thus, the case naturally corresponds to a poor prognosis group (Table 2). In other words, 70% of the poor prognosis group corresponding to at least one of the urine protein excretion rate 0.5 g/g of urinary creatinine or more and eGFR of less than 60 is a poor prognosis group with overt findings.

[Table 2]

| | | Group A | Group B |
|---|---|---|---|
| Total | Number of corresponding cases | 8 cases | 20 cases |
| Urine protein excretion rate of 0.5 g/g of urinary creatinine or more | Number of corresponding cases | 0 cases | 11 cases |
| | Ratio of corresponding cases (%) | 0% | 55% |
| eGFR of less than 60 | Number of corresponding cases | 0 cases | 7 cases |
| | Ratio of corresponding cases (%) | 0% | 35% |
| Urine protein excretion rate of 0.5 g/g of urinary creatinine or more and eGFR of less than 60 | Number of corresponding cases | 0 cases | 14 cases |
| | Ratio of corresponding cases (%) | 0% | 70% |

It is important how to discriminate a poor prognosis group with no overt findings, which accounts for the remaining 30% of the poor prognosis group. Thus, a total of 14 cases (8 cases in a good prognosis group and a relatively good prognosis group and 6 cases in a relatively poor prognosis group and a poor prognosis group) corresponding to eGFR of 60 or more and an urine protein excretion rate of less than 0.5 g/g of creatinine were subjected to subanalysis on prognostic screening effects.

Table 3 shows the results.

[Table 3]

| | PCX/Cre | 1/eGFR | Urine protein /Cre | (PCX/Cre) /eGFR | (PCX/Cre)* (Urine protein/Cre) |
|---|---|---|---|---|---|
| Area under ROC curve | 0.813 | 0.479 | 0.750 | 0.833 | 0.917 |

The results of Table 3 found that "PCX/Cre" itself, "(PCX/Cre)/eGFR," and "(PCX/Cre)*(urine protein/Cre)" each provided a high prognostic screening effect for IgA nephropathy. In particular, the results found that "(PCX/Cre)*(urine protein/Cre)" provided an outstanding prognostic screening effect (FIGS. 1, 2, 3, 4, and 5). Accordingly, it was revealed that prognostic screening for IgA nephropathy and renal biopsy using it as an indicator can be effectively realized with an index value using "PCX/Cre."

More specifically, in cases with overt findings (eGFR of less than 60 or an urine protein excretion rate of 0.5 g/g of creatinine or more), effective screening can be performed with "1/eGFR" or "urine protein/Cre) itself (Table 1 and Table 2), whereas in cases with no overt findings, a poor prognosis group can be discriminated with "PCX/Cre" itself, "(PCX/Cre)/eGFR," and "(PCX/Cre)* (urine protein/Cre) " (Table 3 and FIG. 1, FIG. 2, FIG. 3, FIG. 4, and FIG. 5). Further, as shown in Table 1, also in the cases with overt findings, a screening effect can be obtained with "(PCX/Cre)/eGFR" and "(PCX/Cre)*(urine protein/Cre)." Thus, effective screening for a poor prognosis group can be performed with "(PCX/Cre)/eGFR" and "(PCX/Cre)*(urine protein/Cre)" consistently in general prognostic screening for IgA nephropathy.

In this example, in the cases with overt findings, "PCX/Cre" alone does not necessarily reflect glomerular lesions because complications, i.e., glomerulosclerosis and renal tubular disorder exist. However, the use of an index value using "eGFR" or "urine protein/Cre" in combination therewith allowed the status of active disease progression of glomerular lesions to be evaluated in real time. This indicates that an increase in urinary podocalyxin excretion rate reflects active disease progression in the glomerulus, leading to overt findings.

Industrial Applicability

As described above, the pathology of a renal disease can be grasped by the test method of the present invention. For example, in patients with IgA nephropathy, when the patients are predicted to have relatively poor prognosis and poor prognosis using as an indicator "PCX/Cre" and an index value using it, definitive diagnosis can be performed by further performing renal biopsy. Further, a patient with eGFR of ≥60 and "urine protein/Cre" of less than 0.5 g/g of creatinine can be predicted for its prognosis for IgA nephropathy, and the prognostic prediction is more exact than that of a conventional method. Thus, it is not necessary to perform renal biopsy in a patient with a mild renal disease, which allows a physical burden on the patient and a medical treatment cost to be reduced. Further, when a patient is judged to be in need of renal biopsy, it is possible to make a rapid decision on a therapeutic strategy or the like for the patient, which is useful.

**Claims**

1. A test method for prognostic prediction of IgA nephropathy, comprising calculating a podocalyxin index value using a value for the urinary podocalyxin and a value for the additional marker, and using the podocalyxin index value as an indicator, wherein a value for the additional marker is an estimated glomerular filtration rate (eGFR) or a value for the urine protein excretion rate (urine protein/Cre); wherein the podocalyxin index value is obtained

   by dividing the urinary podocalyxin excretion rate (PCX/Cre) by the estimated glomerular filtration rate (GFR) or
   by multiplying the urinary podocalyxin excretion rate (PCX/Cre) by the value for the urine protein excretion rate (urine protein/Cre),

2. A test method for prognostic prediction of IgA nephropathy according to claim 1, further comprising assessing a subject to be tested who has a podocalyxin index value higher than a podocalyxin reference value to be in need of renal biopsy.

3. A test method for prognostic prediction of IgA nephropathy according to claim 2, wherein the podocalyxin reference value comprises a value obtained from a group of subjects to be tested including a good prognosis group and a relatively good prognosis group of IgA nephropathy, wherein a subject from a good prognosis group is defined

   in that no glomerulosclerosis, crescent formation, and adhesion to the Bowman's capsule are found and in that remarkable renal tubular, interstitial, and vascular changes are not found; and

   wherein a subject from a relatively good prognosis group is defined

   in that glomerulosclerosis, crescent formation, and adhesion to the Bowman's capsule are found in less than 10% of all the glomeruli subjected to biopsy and
   in that remarkable renal tubular, interstitial, and vascular changes are not found.

4. A test method for prognostic prediction of IgA nephropathy according to any one of claims 1 to 3, wherein the test using urinary podocalyxin is performed on specimens, with the exception of specimens that had been assessed to belong to a poor prognosis group with an additional renal function marker, wherein a subject from a poor prognosis group is defined

   in that glomerulosclerosis, crescent formation, and adhesion to the Bowman's capsule are found in 30% or more of all the glomeruli subjected to biopsy and
   in that the sclerosis rate is 50% or more of all the glomeruli, when sites of sclerosis are totaled and converted to global sclerosis

5. A test method for prognostic prediction of IgA nephropathy according to any one of claims 1 to 4, wherein the detecting of the urinary podocalyxin is carried out by an immunological technique.

**Patentansprüche**

1. Testverfahren zur prognostischen Vorhersage von IgA-Nephropathie, umfassend die Berechnung eines Podocalyxin-Indexwerts unter Verwendung eines Werts für das Urin-Podocalyxin und eines Werts für den zusätzlichen Marker und unter Verwendung des Podocalyxin-Indexwerts als einen Indikator, wobei ein Wert für den zusätzlichen Marker eine geschätzte glomeruläre Filtrationsrate (eGFR) oder ein Wert für die Urinprotein-Ausscheidungsrate (Urinprotein/Cre) ist; wobei der Podocalyxin-Indexwert erhalten wird durch

die Division der Urin-Podocalyxin-Ausscheidungsrate (PCX/Cre) durch die geschätzte glomeruläre Filtrationsrate (eGFR) oder

die Multiplizierung der Urin-Podocalyxin-Ausscheidungsrate (PCX/Cre) mit dem Wert für die Urinprotein-Ausscheidungsrate (Urinprotein/Cre).

2. Testverfahren zur prognostischen Vorhersage von IgA-Nephropathie nach Anspruch 1, weiter umfassend die Bewertung eines Subjekts, das getestet werden soll, das einen Podocalyxin-Indexwert aufweist, der höher als ein Podocalyxin-Referenzwert ist, um eine Nierenbiopsie zu erfordern.

3. Testverfahren zur prognostischen Vorhersage von IgA-Nephropathie nach Anspruch 2, wobei der Podocalyxin-Referenzwert einen Wert umfasst, der von einer Gruppe von Subjekten erhalten wird, die getestet werden sollen, darin eingeschlossen eine Gruppe mit einer guten Prognose und eine Gruppe mit einer relativ guten Prognose von IgA-Nephropathie,

wobei ein Subjekt von einer Gruppe mit einer guten Prognose dadurch definiert ist, dass

keine Glomerulosklerose, Sichelbildung und Adhäsion an die Bowman-Kapsel anzutreffen sind, und dadurch, dass bemerkenswerte Nierentubulus-, interstitielle und vaskuläre Änderungen nicht anzutreffen sind; und

wobei ein Subjekt aus einer Gruppe mit einer relativ guten Prognose dadurch definiert ist, dass Glomerulosklerose, Sichelbildung und Adhäsion an die Bowman-Kapsel in weniger als 10 % aller Glomeruli, die einer Biopsie unterzogen sind, anzutreffen sind, und dadurch, dass bemerkenswerte Nierentubulus-, interstitielle und vaskuläre Änderungen nicht anzutreffen sind.

4. Testverfahren zur prognostischen Vorhersage von IgA-Nephropathie nach einem der Ansprüche 1 bis 3, wobei der Test unter Verwendung von Urin-Podocalyxin auf Proben durchgeführt wird, mit der Ausnahme von Proben, bei denen festgestellt wurde, dass sie zu einer Gruppe mit schlechter Prognose mit einem zusätzlichen Marker der Nierenfunktion gehören, wobei ein Subjekt von einer Gruppe mit einer schlechten Prognose dadurch definiert ist, dass

Glomerulosklerose, Sichelbildung und Adhäsion an die Bowman-Kapsel in 30 % oder mehr aller Glomeruli anzutreffen sind, die einer Biopsie unterzogen sind, und dadurch, dass die Skleroserate 50 % oder mehr von allen Glomeruli beträgt, wenn sich Sklerosestellen summieren und in eine globale Sklerose verwandeln.

5. Testverfahren zur prognostischen Vorhersage von IgA-Nephropathie nach einem der Ansprüche 1 bis 4, wobei der Nachweis des Urin-Podocalyxins durch eine immunologische Technik erfolgt.

**Revendications**

1. Un procédé de test pour une prédiction pronostique de néphropathie à IgA, comprenant le calcul d'une valeur d'indice de podocalyxine au moyen d'une valeur de la podocalyxine urinaire et une valeur du marqueur additionnel, et l'utilisation de la valeur d'indice de podocalyxine en tant qu'indicateur,

dans lequel une valeur du marqueur additionnel est un débit de filtration glomérulaire (eGFR) estimé ou une valeur de la vitesse d'excrétion de protéine urinaire (protéine urinaire/Cre),

dans lequel la valeur d'indice de podocalyxine est obtenue

par la division de la vitesse d'excrétion de la podocalyxine urinaire (PCX/Cre) par le débit de filtration glomérulaire estimé (eGFR) ou

par la multiplication de la vitesse d'excrétion de la podocalyxine urinaire (PCX/Cre) avec la valeur de la vitesse d'excrétion de protéine urinaire (protéine urinaire/Cre).

2. Un procédé de test pour une prédiction pronostique de néphropathie à IgA selon la Revendication 1, comprenant en outre l'évaluation d'un sujet à tester qui possède une valeur d'indice de podocalyxine plus élevée qu'une valeur de podocalyxine de référence ayant besoin d'une biopsie rénale.

3. Un procédé de test pour une prédiction pronostique de néphropathie à IgA selon la Revendication 2, où la valeur de podocalyxine de référence comprend une valeur obtenue à partir d'un groupe de sujets à tester comprenant un groupe à bon pronostic et un groupe à relativement bon pronostic de néphropathie à IgA,
Dans lequel un sujet provenant d'un groupe à bon pronostic est défini

en ce qu'aucune glomérulosclérose, formation de croissants ni adhésion à la capsule de Bowman ne sont trouvées et
en ce qu'aucune modification vasculaire, interstitielle et tubulo-rénale remarquable ne sont trouvée, et

dans lequel un sujet provenant d'un groupe à relativement bon pronostic est défini

en ce qu'une glomérulosclérose, une formation de croissants et une adhésion à la capsule de Bowman sont trouvées dans moins de 10% de tous les glomérules soumis à biopsie, et
en ce qu'aucune modification vasculaire, interstitielle et tubulo-rénale remarquable ne sont trouvée.

4. Un procédé de test pour une prédiction pronostique de néphropathie à IgA selon l'une quelconque des Revendications 1 à 3, dans lequel le test au moyen de la podocalyxine urinaire est exécuté sur des spécimens, à l'exception de spécimens qui ont été évalués appartenir à un groupe à mauvais pronostic avec un marqueur de fonction rénale additionnel, dans lequel un sujet provenant d'un groupe à mauvais pronostic est défini

en ce qu'une glomérulosclérose, une formation de croissants et une adhésion à la capsule de Bowman est trouvée dans 30% ou plus de tous les glomérules soumis à biopsie, et
en ce que le taux de sclérose est de 50% ou plus de tous les glomérules, lorsque les sites de sclérose sont totalisés et convertis en sclérose globale,

5. Un procédé de test pour une prédiction pronostique de néphropathie à IgA selon l'une quelconque des Revendications 1 à 4, dans lequel la détection de la podocalyxine urinaire est réalisée au moyen d'une technique immunologique.

[Figure 1]

PCX/Cre

[Figure 2]

(PCX/Cre)/eGFR

[Figure 3]

(PCX/Cre)*(urine protein/Cre)

[Figure 4]

[Figure 5]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002037099 A1
- JP 2592121 B
- JP 2000241431 A

**Non-patent literature cited in the description**

- **HARA et al.** *Nephron,* 1995, vol. 69, 397-403
- **HARA et al.** *Nephrology,* 28 June 2008, vol. 7 (S